# EUROPEAN PATENT SPECIFICATION

(11) **EP 2 364 098 B1**
(45) Date of publication and mention of the grant of the patent: **12.10.2016**
(21) Application number: 09793420.2
(22) Date of filing: 16.12.2009
(51) Int. Cl.: A23K 50/40, A23K 20/121, A61K 31/385

(54) **ANTIOXIDANT-CONTAINING FOOD COMPOSITION FOR USE IN ENHANCING ANTIVIRAL IMMUNITY IN COMPANION ANIMALS**
ANTIOXIDATIONSMITTELHALTIGE NAHRUNGSMITTELZUSAMMENSETZUNG FÜR DIE VERWENDUNG ZUR FÖRDERUNG DER ANTIVIRALEN IMMUNITÄT BEI HAUSTIEREN
COMPOSITION ALIMENTAIRE CONTENANT UN ANTI-OXYDANT CONTRIBUANT À AMÉLIORER L'IMMUNITÉ ANTIVIRALE DES ANIMAUX DE COMPAGNIE

(30) Priority: 16.12.2008 US 122920 P
(43) Date of publication of application: 14.09.2011
(73) Proprietor: Hill's Pet Nutrition, Inc., Topeka, KS 66603 (US)
(72) Inventor: BROCKMAN, Jeffrey A., Lawrence Kansas 66047 (US); FRANTZ, Nolan Zebulon, Topeka Kansas 66618 (US); ZICKER, Steven C., Lawrence Kansas 66049 (US)
(74) Representative: Daniels, Jeffrey Nicholas
(86) International application number: PCT/US2009/068230
(87) International publication number: WO 2010/077933

(56) References cited:
- WO-A2-2006/058248
- PATRICK L: "NUTRIENTS AND HIV: PART THREE - N-ACETYLCYSTEINE, ALPHA-LIPOIC ACID, L-GLUTAMINE, AND L-CARNITINE" ALTERNATIVE MEDICINE REVIEW, THORNE RESEARCH INC., SANDPOINT, US, vol. 5, no. 4, 1 August 2000 (2000-08-01), pages 290-305, XP009004232 ISSN: 1089-5159
- DATABASE MEDLINE [Online] US NATIONAL LIBRARY OF MEDICINE (NLM), BETHESDA, MD, US; 15 October 1999 (1999-10-15), BERKSON B M: "A conservative triple antioxidant approach to the treatment of hepatitis C. Combination of alpha lipoic acid (thioctic acid), silymarin, and selenium: three case histories." XP002567154 Database accession no. NLM10554539 & MEDIZINISCHE KLINIK (MUNICH, GERMANY : 1983) 15 OCT 1999, vol. 94 Suppl 3, 15 October 1999 (1999-10-15), pages 84-89, ISSN: 0723-5003

## Description

### FIELD OF THE INVENTION

The invention encompasses methods for enhancing the ability of a companion animal to resist and/or overcome viral infections. The methods of the invention include an amount of lipoic acid that is effective in enhancing the antiviral immunity of a companion animal.

### BACKGROUND OF THE INVENTION

Companion animals such as dogs and cats seem to suffer from aging problems. Some of these are manifested in commonplace sayings. One of these is "You can't teach an old dog new tricks." This saying arises from the observation that as dogs age, their mental capacity seems to diminish as well as physical abilities. Mental activities associated with thinking, learning and memory seem to be lessened (Cummings, B. J., Head, E., Ruehl, W., Milgram, N. W. & Cotman, C. W. (1996): The canine as an animal model of aging and dementia. Neurobiology of Aging 17:259-268). Additionally, behavioral change can be manifested in the aging animals in association with the changing mental capacity. Many causes have been assigned to this lessening of capacity.

These losses in capacity are generally observed in aged canines and felines. Dogs of seven years or older and felines of seven years or older are considered aged and can experience this problem.

The presence of significant levels of at least one antioxidant in the diet of an adult companion pet or fed to a pet outside his diet can inhibit the onset of deterioration of the mental capacity of the aged companion pet and/or maintain the mental capacity of the adult companion pet further into the aged years.

### SUMMARY OF THE INVENTION

WO2006/058248 discloses methods for increasing the immune response in an animal.

Patrick L (Alternative Medicine Review, Thorn Research Inc., Sandpoint, US, vol 5, no. 4, 2000, pages 290-305, XP009004232) discloses the use of n-acetyl cysteine, alpha-lipoic acid, L-glutamine and L-carnitine in HIV infection.

Berkson BM (Database Accession No. NLM10554539 and Medizinische Klinik 15 October 1999, vol. 94, supplement 3, pages 84-89; XP002567154) discloses a conservative triple antioxidant approach to the treatment of hepatitis C.

### SUMMARY OF THE INVENTION

In a first aspect, the present invention provides a food composition for use in enhancing a companion animal's ability to resist or combat a viral infection, comprising lipoic acid and one or more of vitamin E, vitamin C, beta-carotene, selenium, lutein, tocotrienols, coenzyme Q10, S-adenosylmethioine, taurine, soy isoflavones, N-acetylcysteine, glutathione, and gingko biloba, wherein the lipoic acid is present in an amount of at least 25ppm, wherein the enhancement in the companion animal's ability to resist or combat a viral infection comprises an indication of an interferon-mediated antiviral response.

In a second aspect, the present invention provides a food composition for use in treating a viral infection in a companion animal comprising lipoic acid and one or more of vitamin E, vitamin C, beta-carotene, selenium, lutein, tocotrienols, coenzyme Q10, S-adenosylmethioine, taurine, soy isoflavones, N-acetylcysteine, glutathione, and gingko biloba, wherein the lipoic acid is present in an amount of at least about 25ppm, wherein the treatment of the viral infection comprises an indication of an interferon-mediated antiviral response, and wherein the companion animal is a dog.

### DETAILED DESCRIPTION OF THE INVENTION

### General Description

The invention is directed to a food composition comprising lipoic acid for use in enhancing a companion animal's ability to resist or combat a viral infection. In the invention, the enhancement in the companion animal's ability to resist or combat a viral infection comprises an induction of an interferon-mediated antiviral response.

In the invention, the effective amount of lipoic acid to enhance a companion animal's ability to resist or combat a viral infection is at least 25 ppm.

In certain embodiments, the effective amount is at least 50 ppm.

In certain embodiments, the effective amount is at least 100 ppm.

In certain embodiments, the effective amount is 100 ppm to 600 ppm.

In certain embodiments, the effective amount is 100 ppm to 200 ppm.

In the invention, the companion animal is a dog.

The effective amount is effective to enhance innate antiviral activity in a companion animal.

In certain embodiments, the pet food composition comprising lipoic acid is administered at least 15 days.

In certain embodiments, the pet food composition comprising lipoic acid is administered at least 30 days.

In certain embodiments, the pet food composition comprising lipoic acid is administered at least 45 days.

In certain embodiments, the pet food composition comprising lipoic acid is administered daily.

The invention is further directed to a food composition comprising lipoic acid for use in treating a viral infection. In the invention, the treatment of the viral infection comprises an induction of an interferon-mediated antiviral response.

In the invention, the effective amount of lipoic acid is at least 25 ppm.

In certain embodiments, the effective amount is at least 50 ppm.

In certain embodiments, the effective amount is at least 100 ppm.

In certain embodiments, the effective amount is 100 ppm to 600 ppm.

In certain embodiments, the effective amount is 100 ppm to 200 ppm.

In the invention, the companion animal is a dog.

_______________________________________.

In certain embodiments, the pet food composition comprising lipoic acid is administered at least 15 days.

In certain embodiments, the pet food composition comprising lipoic acid is administered at least 30 days.

In certain embodiments, the pet food composition comprising lipoic acid is administered at least 45 days.

In certain embodiments, the pet food composition comprising lipoic acid is administered daily.

The diet fed to the adult companion canine is the standard normal diet fed to an animal of that age. Below is a typical diet for a canine of 1 to 6 years of age.

**Table 1**

| **Component** | **Target** |
|---|---|
| Protein (% of dry matter) | 23 |
| Fat (% of dry matter) | 15 |
| Phosphorus (% of dry matter) | 0.6 |
| Sodium (% of dry matter) | 0.3 |

The inventors have also surprisingly found that the addition of one or more antioxidants, is useful in enhancing the innate antiviral immune function in dogs.

In the present invention, the food compositions comprise one or more of vitamin E, vitamin C, beta-carotene, selenium, lutein, tocotrienols, coenzyme Q10, S-adenosylmethioine, taurine, soy isoflavones, N-acetylcysteine, glutathione, and gingko biloba. As used herein, the term "enhance" or "enhancing" when referring to antiviral immune function refers to the ability of a companion animals to have an increased immune response to an antigen and thereby be more resistant to infection or clear viral infections from the system of the companion animal faster. Accordingly, a dog, eating a pet food containing an antioxidant, for example, lipoic acid will be more resistant to and will clear viral infections faster than an animal not consuming antioxidants.

The component in the diet which accomplishes this is an antioxidant or mixture thereof. An antioxidant is a material that quenches a free radical. Examples of such materials include foods such as ginkgo biloba, citrus pulp, grape pomace, tomato pomace, carrot and spinach, all preferably dried, as well as various other materials such as beta-carotene, selenium, coenzyme Q10 (ubiquinone), lutein, tocotrienols, soy isoflavones, S-adenosylmethionine, gluthathione, taurine, N-acetylcysteine, vitamin E, vitamin C, alpha-lipoicacid, and L-carnitine. Vitamin E can be administered as a tocopherol or a mixture of tocopherols and various derivatives thereof such as esters like vitamin E acetate, succinate, palmitate, and the like. The alpha form is preferable but beta, gamma and delta forms can be included. The D form is preferable but racemic mixtures are acceptable. The forms and derivatives will function in a Vitamin E like activity after ingestion by the pet. Vitamin C can be administered in this diet as ascorbic acid and its various derivatives thereof such as calcium phosphate salts, cholesteryl salt, 2-monophosphate, which will function in a vitamin C like activity after ingesting by the pet. They can be in any form such as liquid, semisolid, solid and heat stable form. Alpha-lipoic acid can be administered into the diet as alpha-lipoic acid or as a lipoate derivative as in U.S. Pat. No. 5,621,117, racemic mixtures, salts, esters or amides thereof. L- carnitine can be administered in the diet and various derivatives of carnitine such as the salts such as the hydrochloride, fumarate and succinates, as well as acetylated carnitine can be used.

The quantities administered in the diet, all as wt % (dry matter basis) of the diet, are calculated as the active material, per se, that is measured as free material. The maximum amounts employed should not bring about toxicity.

At least about 100 ppm or at least about 150 ppm of vitamin E can be used. In certain embodiments, the range of about 500 to about 1,000 ppm can be employed. Although not necessary a maximum of about 2,000 ppm or about 1,500 ppm is generally not exceeded.

With respect to vitamin C at least about 50 ppm is used, desirably at least about 75 ppm and more desirably at least about 100 ppm. A nontoxic maximum can be employed.

The quantity of alpha-lipoic acid can vary from at least about 25, desirably at least 50 ppm, more desirably 100 ppm. In various embodiments, the range of lipoic acid that can be administered dogs is 150 ppm to 4500 ppm. Maximum quantities can vary from about 100 ppm to 600 ppm or to an amount which remains nontoxic to the pet. In certain embodiments, a range is from about 100 ppm to about 200 ppm.

For L-carnitine about 50 ppm, desirably about 200 ppm, more desirably about 300 ppm for canines are a useful minimum. A nontoxic maximum quantity can be employed, for example, less than about 5,000 ppm. For canines, lower quantities can be employed, for example, less than about 5,000 ppm. For canines a preferred range is about 200 ppm to about 400 ppm.

Beta-carotene at about 1-15 ppm can be employed.

Selenium at about 0.1 up to about 5 ppm can be employed.

Lutein: at least about 5 pm can be employed.

Tocotrienols: at least about 25 ppm can be employed.

Coenzyme Q10: at least about 25 ppm can be employed.

S-adenosylmethionine: at least about 50 ppm can be employed.

Taurine: at least about 1000 ppm can be employed.

Soy isoflavones: at least about 25 ppm can be used.

N-acetylcysteine: at least about 50 ppm can be used.

Glutathione: at least about 50 ppm can be used.

Gingko biloba: at least 50 ppm of extract can be used.

The following are raw ingredients that are high in ORAC (Oxygen radical absorbing capacity) content: Spinach pomace, Tomato pomace, Citrus pulp, Grape pomace, Carrot granules, Broccoli, Green tea, Ginkgo biloba and Corn gluten meal. When added to the diet as 1% inclusions (for a total of 5% substitution for a low ORAC ingredient such as corn) they increased the ORAC content of the overall diet and increased the ORAC content of the plasma of the animals which ate the diet containing these components. Preferably, any ingredient with an ORAC content >25 .mu.mole of Trolox equivalents per gram of dry matter could be used if added at 1% in combination with four other 1% ingredients for a total of 5% addition to the diet.

### Example 1

### Experimental Conditions

Twenty dogs were fed for 30 days. Ten were fed an AAFCO level control food and 10 other dogs were fed the AAFCO level control food containing 150 ppm alpha-lipoic acid. At the end of the end of the 30 days whole blood samples were collected from each dog in Paxgene tubes.

Total RNAs were isolated from whole blood samples using the PAXgene RNA isolation kit. All measurements were done with the canine 2 Affymetrix genechips. For statistical analysis, all measurements were normalized with RMA. All analysis was preformed using Partek. An ANOVA t-test was performed for genes that are differentially expressed between the control and test foods. (at least a 20% change in expression with a pvalue < 0.05)

Differentially expressed genes were analyzed with the GeneGo pathway analysis software. Dogs fed lipoic acid for 30 days exhibited an interferon mediated antiviral response. Genes up-regulated by feeding dogs lipoic acid for 30 days that are involved in interferon mediated antiviral response are listed in Table 4.

**Table 1**

| | | | **30-days lipoic acid canine adult** | |
|---|---|---|---|---|
| **Gene Symbol** | **Protein** | **Protein Name** | **Fold Up-regulated** | **p-value** |
| CREBBP | CBP Human | CREB-binding protein | 1.2 | 0.04 |
| EIF2AK2 | E2AK2 Human | Interferon-induced double stranded RNA-activated protein | 1.4 | 0.04 |
| IFNAR2 | INAR2 Human | interferon-alpha/beta receptor beta chain precursor | 1.3 | 0.01 |
| IFNGR2 | INGR2 Human | interferon-gamma receptor beta chain precursor | 1.2 | 0.03 |
| IRF9 | IRF9 Human | interferon regulatory factor 9 | 1.3 | 0.2 |
| JAK2 | JAK2 Human | Tyrosine protein kinase JAK2 | 1.4 | 0.01 |
| RNASEL | RN5A Human | 2-5A-dependent ribonuclease | 1.5 | 0.04 |

Based on the studies of dogs fed lipoic acid for 30 days, the inventors have surprisingly found that cell surface receptors for interferon alpha/beta and interferon gamma are increased leading to the potential for increasing the entire interferon mediated antiviral defense mechanism. The inventors have found that JAK2, a key activator of STAT1 and STAT2, is up regulated. Interferon regulatory factor 9 (IFR9) is up-regulated. IFR9, STAT1 and STAT2 form a complex (ISFG3) that translocates to the nucleus and up regulates the antiviral genes, interferon-induced, double stranded RNA-activated protein kinase (PKR) and 2-5A-dependent ribonuclease (RnaseL). PKR inhibits elF2S1 via phosphorylation leading to an inhibition of viral protein synthesis. RnaseL cleaves viral RNA inhibiting viral replication and function.

## Claims

1. A food composition for use in enhancing a companion animal's ability to resist or combat a viral infection, comprising lipoic acid and one or more of vitamin E, vitamin C, beta-carotene, selenium, lutein, tocotrienols, coenzyme Q10, S-adenosylmethioine, taurine, soy isoflavones, N-acetylcysteine, glutathione, and gingko biloba,
wherein the lipoic acid is present in an amount of at least 25ppm,
wherein the enhancement in the companion animal's ability to resist or combat a viral infection comprises an induction of an interferon-mediated antiviral response, and wherein the companion animal is a dog.

2. A food composition for use in treating a viral infection in a companion animal comprising lipoic acid and one or more of vitamin E, vitamin C, beta-carotene, selenium, lutein, tocotrienols, coenzyme Q10, S-adenosylmethioine, taurine, soy isoflavones, N-acetylcysteine, glutathione, and gingko biloba,
wherein the lipoic acid is present in an amount of at least 25ppm, wherein the treatment of the viral infection comprises an induction of an interferon-mediated antiviral response, and
wherein the companion animal is a dog.

3. A food composition for use according to claim 1 or claim 2, wherein the lipoic acid is present in an amount of at least 50 ppm.

4. A food composition for use according to any preceding claim, wherein the lipoic acid is present in an amount of at least 100 ppm.

5. A food composition for use according to any preceding claim, wherein the lipoic acid is present in an amount of at least 100 ppm to 600 ppm.

6. A food composition for use according to any one preceding claim, wherein the lipoic acid is present in an amount of at least 100 ppm to 200 ppm.

7. A food composition for use according to any preceding claim, wherein the composition enhances innate antiviral activity in a companion animal.

8. A food composition for use according to any preceding claim, wherein the composition comprising lipoic acid is administered at least 15 days.

9. A food composition for use according to any preceding claim, wherein the food composition comprising lipoic acid is administered at least 30 days.

10. A food composition for use according to any preceding claim, wherein the pet food composition comprising lipoic acid is administered at least 45 days.

11. A food composition for use according to any preceding claim, wherein the pet food composition comprising lipoic acid is administered to the companion animal daily.

## Patentansprüche

1. Futterzusammensetzung zur Verwendung bei der Verstärkung der Fähigkeit eines Haustiers, einer Virusinfektion standzuhalten oder eine Virusinfektion zu bekämpfen, wobei die Futterzusammensetzung Liponsäure und eines oder mehrere von Vitamin E, Vitamin C, Betacarotin, Selen, Lutein, Tocotrienolen, Coenzym Q10, S-Adenosylmethionin, Taurin, Soja-Isoflavonen, N-Acetylcystein, Glutathion und Ginkgo biloba umfasst,
wobei die Liponsäure in einer Menge von mindestens 25 ppm vorliegt,
wobei die Verstärkung der Fähigkeit eines Haustiers, einer Virusinfektion standzuhalten oder eine Virusinfektion zu bekämpfen, ein Induzieren einer von Interferon vermittelten antiviralen Reaktion umfasst, und wobei das Haustier ein Hund ist.

2. Futterzusammensetzung zur Verwendung bei der Behandlung einer Virusinfektion bei einem Haustier, wobei die Futterzusammensetzung Liponsäure und eines oder mehrere von Vitamin E, Vitamin C, Betacarotin, Selen, Lutein, Tocotrienolen, Coenzym Q10, S-Adenosylmethionin, Taurin, Soja-Isoflavonen, N-Acetylcystein, Glutathion und Ginkgo biloba umfasst,
wobei die Liponsäure in einer Menge von mindestens 25 ppm vorliegt, wobei die Behandlung der Virusinfektion ein Induzieren einer von Interferon vermittelten antiviralen Reaktion umfasst und wobei das Haustier ein Hund ist.

3. Futterzusammensetzung zur Verwendung nach Anspruch 1 oder 2, wobei die Liponsäure in einer Menge von mindestens 50 ppm vorliegt.

4. Futterzusammensetzung zur Verwendung nach einem vorhergehenden Anspruch, wobei die Liponsäure in einer Menge von mindestens 100 ppm vorliegt.

5. Futterzusammensetzung zur Verwendung nach einem vorhergehenden Anspruch, wobei die Liponsäure in einer Menge von mindestens 100 ppm bis 600 ppm vorliegt.

6. Futterzusammensetzung zur Verwendung nach einem vorhergehenden Anspruch, wobei die Liponsäure in einer Menge von mindestens 100 ppm bis 200 ppm vorliegt.

7. Futterzusammensetzung zur Verwendung nach einem vorhergehenden Anspruch, wobei die Zusammensetzung eine angeborene antivirale Aktivität in einem Haustier verstärkt.

8. Futterzusammensetzung zur Verwendung nach einem vorhergehenden Anspruch, wobei die Zusammensetzung, die Liponsäure umfasst, für mindestens 15 Tage verabreicht wird.

9. Futterzusammensetzung zur Verwendung nach einem vorhergehenden Anspruch, wobei die Futterzusammensetzung, die Liponsäure umfasst, für mindestens 30 Tage verabreicht wird.

10. Futterzusammensetzung zur Verwendung nach einem vorhergehenden Anspruch, wobei die Tierfutterzusammensetzung, die Liponsäure umfasst, für mindestens 45 Tage verabreicht wird.

11. Futterzusammensetzung zur Verwendung nach einem vorhergehenden Anspruch, wobei die Tierfutterzusammensetzung, die Liponsäure umfasst, dem Haustier täglich verabreicht wird.

## Revendications

1. Une composition alimentaire destinée à être utilisée pour améliorer la capacité d'un animal de compagnie à résister à, ou à lutter contre, une infection virale, comprenant l'acide lipoïque et un ou plusieurs éléments parmi la vitamine E, la vitamine C, la bêta-carotène, le sélénium, la lutéine, les tocotriénols, le coenzyme Q10, la S-adenosylméthionine, la taurine, les isoflavones de soja, la N-acétylcystéine, le glutathion et le gingko biloba,
dans laquelle l'acide lipoïque est présent dans une quantité d'au moins 25 ppm,
dans laquelle l'amélioration de la capacité d'un animal de compagnie à résister à, ou à lutter contre, une infection virale comprend une induction d'une réponse antivirale induite par l'interféron, et
dans laquelle l'animal de compagnie est un chien.

2. Une composition alimentaire destinée à être utilisée dans le traitement d'une infection virale chez un animal de compagnie, comprenant l'acide lipoïque et un ou plusieurs éléments parmi la vitamine E, la vitamine C, la bêta-carotène, le sélénium, la lutéine, les tocotriénols, le coenzyme Q10, la S-adenosylméthionine, la taurine, les isoflavones de soja, la N-acétylcystéine, le glutathion et le gingko biloba,
dans laquelle l'acide lipoïque est présent dans une quantité d'au moins 25 ppm,
dans laquelle le traitement de l'infection virale comprend une induction d'une réponse antivirale induite par l'interféron, et
dans laquelle l'animal de compagnie est un chien.

3. Une composition alimentaire destinée à être utilisée selon la revendication 1 ou la revendication 2, dans laquelle l'acide lipoïque est présent dans une quantité d'au moins 50 ppm.

4. Une composition alimentaire destinée à être utilisée selon l'une quelconque des revendications précédentes, dans laquelle l'acide lipoïque est présent dans une quantité d'au moins 100 ppm.

5. Une composition alimentaire destinée à être utilisée selon l'une quelconque des revendications précédentes, dans laquelle l'acide lipoïque est présent dans une quantité d'au moins 100 ppm à 600 ppm.

6. Une composition alimentaire destinée à être utilisée selon l'une quelconque des revendications précédentes, dans laquelle l'acide lipoïque est présent dans une quantité d'au moins 100 ppm à 200 ppm.

7. Une composition alimentaire destinée à être utilisée selon l'une quelconque des revendications précédentes, dans laquelle la composition améliore l'activité antivirale innée chez un animal de compagnie.

8. Une composition alimentaire destinée à être utilisée selon l'une quelconque des revendications précédentes, dans laquelle la composition comprenant l'acide lipoïque est administrée pendant au moins 15 jours.

9. Une composition alimentaire destinée à être utilisée selon l'une quelconque des revendications précédentes, dans laquelle la composition alimentaire comprenant l'acide lipoïque est administrée pendant au moins 30 jours.

10. Une composition alimentaire destinée à être utilisée selon l'une quelconque des revendications précédentes, dans laquelle la composition alimentaire pour animal de compagnie comprenant l'acide lipoïque est administrée pendant au moins 45 jours.

11. Une composition alimentaire destinée à être utilisée selon l'une quelconque des revendications précédentes, dans laquelle la composition alimentaire pour animal de compagnie comprenant l'acide lipoïque est administrée à l'animal de compagnie quotidiennement.
